# EUROPÄISCHE PATENTSCHRIFT

(11) **EP 1 309 561 B1**
(45) Veröffentlichungstag und Bekanntmachung des Hinweises auf die Patenterteilung: **11.10.2006**
(21) Anmeldenummer: 01971875.8
(22) Anmeldetag: 06.08.2001
(51) Int. Cl.: C07D 231/12, C07D 231/16, C05G 3/08

(54) **1H-PYRAZOL-DERIVATE, VERFAHREN ZU IHRER HERSTELLUNG, UND IHRE VERWENDUNG ALS NITRIFIKATIONSINHIBITOREN**
1H-PYRAZOL DERIVATIVES, METHOD FOR PRODUCING THEM AND THEIR USE AS NITRIFICATION INHIBITORS
DERIVES DE 1H-PYRAZOLE, LEURS PROCEDES DE PREPARATION ET LEUR UTILISATION COMME INHIBITEURS DE NITRIFICATION

(30) Priorität: 08.08.2000 DE 10038608
(43) Veröffentlichungstag der Anmeldung: 14.05.2003
(73) Patentinhaber: SKW STICKSTOFFWERKE PIESTERITZ GmbH, 06886 Lutherstadt Wittenberg (DE)
(72) Erfinder: RADICS, Ute, 06888 Dabrun (DE); NICLAS, Hans-Joachim, 12435 Berlin (DE); MICHEL, Hans-Jürgen, 04827 Machern (DE); KRISTOF, Wolfgang, 06886 Wittenberg (DE); WOZNIAK, Hartmut, 04451 Cunnersdorf (DE)
(74) Vertreter: Schneider, Michael
(86) Internationale Anmeldenummer: PCT/EP2001/009074
(87) Internationale Veröffentlichungsnummer: WO 2002/012197

(56) Entgegenhaltungen:
- WO-A-96/24566
- DE-A- 19 849 496
- DE-A- 19 913 475

## Beschreibung

Die vorliegende Erfindung betrifft 1H-Pyrazol-Derivate, Verfahren zu ihrer Herstellung, Düngemittelzusammensetzungen, die sie umfassen, sowie ihre Verwendung zur Hemmung bzw. Steuerung der Nitrifikation.

Die Anwendung von Stickstoffdüngemitteln, die Nitrifikationsinhibitoren enthalten, ist dadurch besonders vorteilhaft, dass die im allgemeinen rasch ablaufende Nitrifikation, d. h. die mikrobielle Umwandlung von Amid- und Ammoniumstickstoff zu Nitratstickstoff, auf diese Weise verzögert wird. Die derart gesteuerte Nitratbildung im Boden vermeidet eine zu intensive Nitratbildung und damit die Akkumulation von Nitrat. Eine so bewirkte Zurückdrängung des sonst entstehenden Nitratüberschusses im Boden konserviert den Düngerstickstoff in Form von Ammoniumionen, sorgt damit für eine höhere Düngereffizienz und führt gleichzeitig zu einer erheblich verringerten Umweltbelastung im Rahmen einer Stickstoffdüngung.

So ist bekannt, dass Nitrat im Gegensatz zum Ammonium im Boden nur äußerst schlecht sorbiert wird und demzufolge der Auswaschung bzw. der Verlagerung in tiefere Boden- schichten unterliegt. Andererseits führt die infolge einer Nitratanreicherung im Boden verstärkt einsetzende Denitrifikation zu gasförmigen Stickstoffverlusten, wobei neben molekularem Stickstoff auch solche Oxide wie Stickstoffmonoxid bzw. Lachgas emittiert werden, die zur Versauerung des Bodens beitragen bzw. als klimaveränderndes Gas bekannt sind. Gleichzeitig wird einer Nitratanreicherung im Grundwasser entgegengewirkt.

Gerade in der Reduzierung der beschriebenen Verlustpotentiale liegt der wirtschaftliche Vorteil von mit Nitrifikationsinhibitoren versehenen Stickstoffdüngemitteln, der sich in der erhöhten Nährstoffeffizienz dieser Dünger niederschlägt. Um das zu unterstreichen sei erwähnt, dass N-Verluste von konventionellen Stickstoffdüngern durchaus unter ungünstigen Bedingungen Größenordnungen von bis zu 40 % und mehr annehmen können.

Als wirksame Nitrifikationshemmer wurden unter anderem stickstoffhaltige heterocyclische Verbindungen, bevorzugt aus der Reihe der 1H-Pyrazole und der 1,2,4-Triazole, vorgeschlagen, z. B. in US 3,635,690, US 4,969,946, US 4,523,940, EP-A 166 420, JP-OS 71-04135, US 3,697,244.

Ein wesentlicher Nachteil der meisten sehr gut nitrifikationshemmend wirkenden Pyrazolderivate besteht in ihrer relativ hohen Flüchtigkeit bzw. besonders bei am N-1-Stickstoffatom substituierten Verbindungen in ihrer Hydrolyseempfindlichkeit, in deren Folge zwar noch wirksame, aber wieder flüchtige Derivate gebildet werden. Aus diesen Gründen lässt sich eine Anwendung bei festen Düngemitteln, insbesondere in Kombinationen mit Harnstoff, nicht ohne weiteres realisieren. Zur Beseitigung dieses Mangels sind Vorschläge zur chemischen Abwandlung der 1-N-unsubstituierten Basispyrazole bekannt (DE-OS 27 45 833, EP-A 508 191, DD 292 232, DE-OS 40 18 395, EP-A 160 804, DE-OS 37 04 359, SU 1470 732, DE-OS 44 46 194, EP-A 808 297). Mit dem Rückgang der Flüchtigkeit durch Derivatisierung ist jedoch in der Regel zugleich auch ein Abfall in der Wirkung als Nitrifikationsinhibitor verbunden.

Andere Lösungen zur Reduzierung der Flüchtigkeit von nitrifikationshemmend wirkenden Pyrazolen bestehen in der Ausnutzung des eigentlich bekannten Prinzips der Salzbildung mit Säuren (EP-A 529 473, WO 98/05 607, DE-OS 4018 395).

Sowohl die mineralsauren Salze der nitrifiziden Basispyrazole als auch nitrifizid wirkende Pyrazole, die am 1-N-Stickstoff hydrophile Gruppen tragen, können als Nitrifikationsinhibitoren noch nicht voll befriedigen, da sie eine zu geringe Hydrolysestabilität besitzen, wodurch die Wirkungsdauer im Boden und die Lagerstabilität herabgesetzt ist. Dies betrifft z. B. N-Glyoxylpyrazole (DE-A 37 04 359) oder N-Hydroxymethylpyrazole (SU-A 1 470 737).

Der vorliegenden Erfindung lag die Aufgabe zugrunde, Wirkstoffe zur Hemmung bzw. Steuerung der Nitrifikation von Ammoniumstickstoff zu entwickeln, die eine ausreichende Residualwirkung aufweisen und die aufgrund ihrer geringen Flüchtigkeit und ihrer Hydrolysestabilität alle Applikationsverfahren, aber auch alle Möglichkeiten zur Einarbeitung in oder Aufbringung auf mineralische stickstoffhaltige Dünger zulassen.

Diese Aufgabe wurde erfindungsgemäß durch die 1H-Pyrazol-Derivate entsprechend Anspruch 1 gelöst.

Es hat sich nämlich überraschenderweise gezeigt, dass diese Verbindungen neben der guten nitrifikationshemmenden Wirkung den außerordentlichen Vorteil einer sehr geringen Hydrolyseneigung besitzen, wodurch Wirkstoffverluste bei der Oberflächenapplikation, aber auch bei der Lagerung von Dünger-Wirkstoff-Gemischen nahezu ausgeschlossen sind.

Außerdem sind die erfindungsgemäßen 1H-Pyrazol-Derivate durch ihre Öllöslichkeit und dem damit verbundenen hydrophoben Charakter für eine Aufbringung auf die Düngeroberfläche sehr gut geeignet, was ebenfalls nicht vorhersehbar war.

Die erfindungsgemäßen 1H-Pyrazol-Derivate entsprechen der allgemeinen Formel (I) 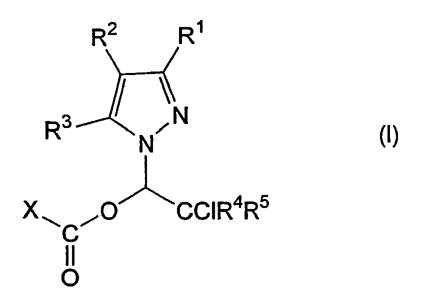 wobei R¹ und R³ unabhängig voneinander für H oder einen C₁-C₄₋Alkylrest, R² für H, Halogen (F, Cl, Br, I) oder einen C₁-C₄₋Alkylrest sowie R⁴ und R⁵ unabhängig voneinander für H oder Cl stehen.

Die C₁- bis C₄-Alkylreste R¹, R² und R³ können linear oder verzweigt sein oder einen Cyclopropyl- oder Cyclobutylrest darstellen. R¹ und R² können auch einen fünf- oder sechsgliedrigen Cycloalkylrest ausbilden. Vorzugsweise stellen R¹ und R² einen Methylrest oder R¹ Methyl und R² Wasserstoff oder Chlor dar.

X steht für OR⁶ oder NHR⁷. Dabei steht R⁶ für einen C₁-C₄₋Alkylrest, einen C₆-C₁₀-Arylrest oder einen Alkylarylrest mit C₁-C₄-Alkylgruppen und C₆-C₁₀-Aryl, und R⁷ steht für einen C₁-C₈-Alkylrest oder einen C₆-C₁₀-Arylrest . Als C₁-C₄₋Alkylreste und als C₁-C₈-Reste kommen lineare oder verzweigte Alkylreste in Frage.

Bevorzugt steht X für O(C₁-C₄-Alkyl) oder für NH (C₁-C₈-Alkyl) . Vorzugsweise steht X für O-Ethyl.

Die erfindungsgemäß vorgeschlagenen 1H-Pyrazol-Derivate sind zugänglich aus den 2-chlorsubstituierten 1-Pyrazolyl-ethanol-Derivaten (IV). Diese Ausgangsverbindungen (IV) bzw. deren Herstellung sind aus der DE-Anmeldung 199 13 475.8 bekannt.

Zur Herstellung der 2-chlorsubstituierten 1-Pyrazolyl-ethanol-Derivate (IV) werden die 1-unsubstituierten Basispyrazole (II) (wobei R¹, R² und R³ oben genannte Bedeutung besitzen) zunächst mit Aldehyden der allgemeinen Formel (III) (wobei R⁴ und R⁵ die oben genannte Bedeutung besitzen) gemäß der allgemeinen Gleichung (1) umgesetzt, wobei das entsprechende Pyrazol mit dem Aldehyd im Molverhältnis 1 : 1,0 bis 1 : 1,1 in Gegenwart eines organischen Lösemittels (vorzugsweise ausgewählt aus der Gruppe Halogenkohlenwasserstoffe, wie z.B. Methylenchlorid oder cyclische Ether wie z. B. Tetrahydrofuran, oder aromatische Kohlenwasserstoffe wie z. B. Toluol) bei Temperaturen von 50 bis 80 °C zur Reaktion gebracht wird.

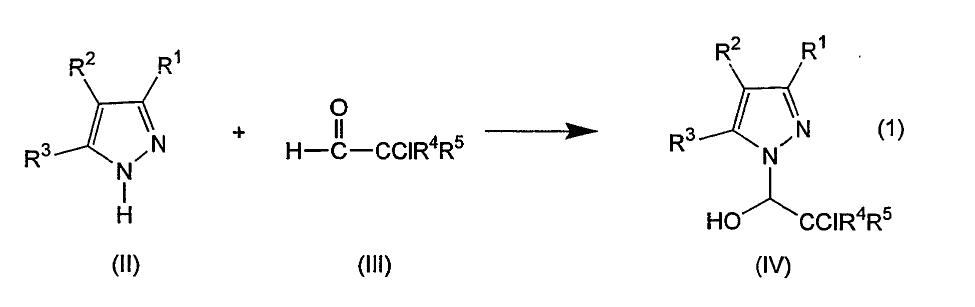

Die erfindungsgemäß vorgeschlagenen 1H-Pyrazol-Derivate der Formel (I) mit X = OR⁶, mit den für R⁶ bereits ausgewiesenen Alkyl-, Aryl- bzw. Alkylarylresten, sind zugänglich indem man die entsprechenden 2-chlorsubstituierten 1-Pyrazolyl-ethanol-Derivat der allgemeinen Formel (IV) wobei R¹, R², R³, R⁴ und R⁵ oben genannte Bedeutung besitzen, entweder direkt oder gegebenenfalls nach deren Isolierung mit dem entsprechenden Chlorameisensäureestern (V) in einem organischen Lösemittel unter Zusatz von organischen Basen gemäß der allgemeinen Gleichung (2) reagieren lässt. Bevorzugt wird die Umsetzung mit den Chlorameisensäureestern in einem organischen Lösemittel ausgewählt aus der Gruppe Halogenkohlenwasserstoffe wie z.B. Methylenchlorid, cyclische Ether wie z.B. Tetrahydrofuran, oder aromatische Kohlenwasserstoffe wie z.B. Toluol unter Zusatz von molaren Mengen an organischer Base mit einem Molverhältnis von 1-Pyrazolyl-ethanol-Derivat (IV) zu Chlorameisensäureester von 1 : 1,0 bis 1,3 und einer Temperatur von -25 bis +10 °C, bevorzugt -25 bis -10 °C, durchgeführt.

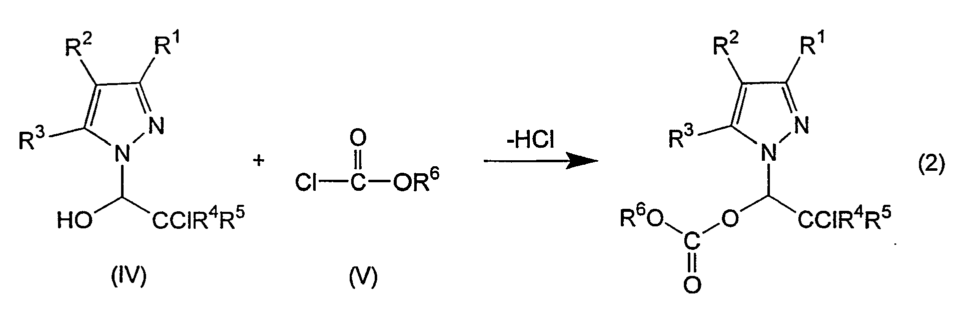

Die erfindungsgemäß vorgeschlagenen 1H-Pyrazol-Derivate mit X = NHR⁷, mit den für R⁷ bereits ausgewiesenen Alkyl- bzw. Arylresten, werden aus den jeweiligen 2-chlorsubstituierten 1-Pyrazolyl-ethanol-Derivaten (IV) durch die Umsetzung mit aliphatischen oder aromatischen Isocyanaten R⁷N=C=O in einem organischen Lösemittel unter Zusatz von organischen Basen gemäß der allgemeinen Gleichung (3) hergestellt. Bevorzugt wird die Umsetzung mit den Isocyanaten in einem organischen Lösemittel ausgewählt aus der Gruppe Carbonsäureester oder aromatische Kohlenwasserstoffe unter Zusatz von 5 bis 10 Mol-% an organischer Base mit einem Molverhältnis von 1-Pyrazolyl-ethanol-Derivat zu Isocyanat von 1 : 1,1 bis 1,3 und einer Temperatur von 40 bis 100 °C vorgenommen.

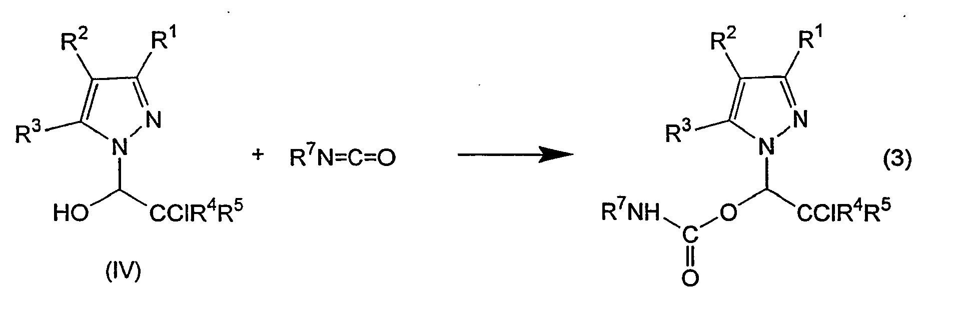

Die Reaktionsprodukte sind in den meisten Fällen destillierbare Flüssigkeiten, die mit zunehmender Molekülgröße viskoser werden.

Bei der Synthese der erfindungsgemäßen Verbindungen entsprechend Gleichung (1) bis (3) wurde das Auftreten von Isomeren in Bezug auf die Stellung des R¹- und R³⁻Substituenten außer Acht gelassen. Es wurde von vornherein von regioisomeren Mischungen der 2-chlorsubstituierten 1-Pyrazolyl-ethanol-Derivate ausgegangen. Die Bildung von optischen Isomeren wurde bei der Herstellung der 2-chlorsubstituierten 1-Pyrazolyl-ethanol-Derivate ebenfalls außer Acht gelassen. Falls gewünscht können die jeweiligen Isomeren jedoch mittels den üblichen, dem Fachmann bekannten Verfahren getrennt werden.

Ein weiterer Gegenstand der vorliegenden Erfindung ist die Verwendung der erfindungsgemäßen 1H-Pyrazol-Derivate zur Hemmung bzw. Regelung der Nitrifikation, wobei die erfindungsgemäß vorgeschlagenen Verbindungen in bekannter Weise als Nitrifikationsinhibitoren mit festen ammonium-und/oder amidhaltigen Düngemitteln kombinierbar sind. Als Stickstoffdüngemittel werden vorzugsweise Ammoniumsalze, wie z. B. Kalkammonsalpeter, Ammoniumsulfatsalpeter, Ammoniumnitrat oder Harnstoff eingesetzt. Möglich ist auch der Einsatz der erfindungsgemäßen 1H-Pyrazol-Derivate in Kombination mit organischen und flüssigen Düngemitteln. Aufwandmengen von 0,01 bis 10,0 Gewichtsprozent, bevorzugt 0,1 bis 5,0 Gewichtsprozent, insbesondere 0,3 bis 3,0 Gewichtsprozent, berechnet auf den reduzierten Stickstoffgehalt des Düngers, d. h. auf Ammonium- und Carbamid-Stickstoff, genügen für den praktischen Einsatz. Zusammensetzungen umfassend die erfindungsgemäßen 1H-Pyrazol-Derivate sowie gegebenenfalls weitere übliche Nitrifikationsinhibitoren und/oder Ureasehemmstoffe sind ein weiterer Gegenstand der Erfindung.

Die Art und Weise, wie die erfindungsgemäß vorgeschlagenen Nitrifikationsinhibitoren mit dem Düngemittel kombiniert werden, ist relativ unkritisch. So können die 1H-Pyrazol-Derivate bevorzugt allein oder im Gemisch mit anderen Zusatzstoffen als Lösung auf die Oberfläche der Düngemittelgranulate aufgebracht werden, wobei das Lösemittel (vorzugsweise aprotische Lösemittel, wie z. B. Ester, Öle, Ketone, Ether etc.) durch Trocknung entfernt wird.

Alternativ hierzu können die 1H-Pyrazol-Derivate allein oder im Gemisch mit anderen Zusatzstoffen in die Düngemittelschmelze oder -slurry während des Formgebungsprozesses eingebracht werden.

Die erfindungsgemäßen Verbindungen zeichnen sich durch ihren lipophilen Charakter aus. Die Einführung des Restes -C(O)X führt zu einem erheblichen Anstieg der Hydrolysestabilität der Verbindungen, ohne dass ein Rückgang der nitrifiziden Wirkung erfolgt. Aufgrund dieser Eigenschaften sind die erfindungsgemäßen 1H-Pyrazol-Derivate bevorzugt für eine Oberflächenapplikation auf den Düngemittelgranulaten geeignet.

Die nachfolgenden Beispiele sollen die Erfindung näher veranschaulichen.

### Beispiele

### Darstellung von 1H-Pyrazol-Derivaten mit X = OR⁶

### Variante A

### Allgemeine Synthesevorschrift:

1 mol Pyrazol (II) wird in 400 ml Methylenchlorid vorgelegt. Unter Rühren und Kühlung tropft man 1 mol Aldehyd (III) so zu, dass die Temperatur nicht über 37 °C steigt. Die Reaktionsmischung wird noch zwei Stunden unter Rückfluss erhitzt und anschließend auf Raumtemperatur abgekühlt. Die Produktlösung (gegebenenfalls muss noch etwas Methylenchlorid nachdosiert werden, um ausgefallenes Produkt zu lösen) wird bei -10 bis +10 °C gleichzeitig mit 1 mol Triethylamin in 200 ml Dichlormethan zu 1,1 mol Chlorameisensäureester in 200 ml Dichlormethan getropft. Man lässt eine Stunde bei dieser Temperatur nachrühren und belässt das Reaktionsgemisch über Nacht bei Raumtemperatur. Das Triethylaminhydrochlorid wird abgesaugt, das Filtrat mit 10 %iger Kochsalzlösung gewaschen, getrocknet und unter Vakuum eingeengt. Das verbleibende Rohprodukt wird durch Säulenchromatographie gereinigt.

### Variante B

### Allgemeine Synthesevorschrift:

0,11 mol Chlorameisensäureester werden in 20 ml Tetrahydrofuran unter Stickstoff vorgelegt und bei -10 °C gleichzeitig 0,1 mol 2-chlorsubstituiertes 1-Pyrazolyl-ethanol-Derivat (IV) in 100 ml THF gelöst und 0,1 mol Triethylamin in 50 ml THF zugetropft. Man lässt eine Stunde bei dieser Temperatur nachrühren und belässt das Reaktionsgemisch über Nacht bei Raumtemperatur. Das Triethylaminhydrochlorid wird abgesaugt und das Filtrat unter Vakuum eingeengt. Der Rückstand wird mit 100 ml Essigsäureethylester aufgenommen, mit 10 %iger Kochsalzlösung gewaschen und getrocknet. Die organische Phase wird zur Trockne eingedampft und das verbleibende Rohprodukt durch Säulenchromatographie gereinigt.

### Darstellung von 1H-Pyrazol-Derivaten mit X = NHR⁷

### Allgemeine Synthesevorschrift:

0,1 mol 2-chlorsubstituiertes 1-Pyrazolyl-ethanol-Derivat (IV) werden in 250 ml Essigsäureethylester gelöst und 1 ml Triethylamin (7,17 mmol) zugegeben. Anschließend werden 0,12 mol Isocyanat in 50 ml Essigsäureethylester innerhalb von 30 Minuten zugetropft. Das Reaktionsgemisch wird drei Stunden unter Rückfluss erhitzt und nach dem Abkühlen aufgearbeitet.

Die Reaktionslösung wird mit 10 %iger Kochsalzlösung gewaschen und getrocknet. Die organische Phase wird zur Trockne eingedampft und das verbleibende Rohprodukt durch Säulenchromatographie gereinigt.

Die in Tabelle 1 aufgeführten Substanzen wurden nach obigen Synthesevarianten erhalten. Die Strukturen wurden durch NMR-Spektroskopie und Elementaranalyse gesichert.

**Tabelle 1: 1H-Pyrazol-Derivate der allgemeinen Formel I**

| **Nr.** | **R**^{**1**} | **R**^{**2**} | **R**^{**3**} | **R**^{**4**}**, R**^{**5**} | **X** | **Ausb.** | **Kp (°C)** | ^{**1**}**H-NMR (CDCl**_{**3**}**)** |
|---|---|---|---|---|---|---|---|---|
| | | | | | | **(%)** | | **δ [ppm]** |
| 1 | CH₃ | H | H | Cl | OC₂H₅ | 67 | 103 | 1,28 (m, 1,22 H) ; 1,40 (m, 1,78 H); |
| | | | | | | (A) | (20 Torr) | 2,26 (d, 1,78 H); 2,51 (d, 1,22 H); |
| | | | | | | | | 4,22 (m, 0,77 H); 4,42 (m, 1,23 H); |
| | | | | | | | | 6,09 (m, 1 H); 6,98 (s, 0,44 H); |
| | | | | | | | | 7,51 (s, 0,56 H); 7,71 (s, 0,52 H); |
| | | | | | | | | 7,99 (s, 0,48 H) |
| 2 | CH₃ | Cl | H | H | OC₂H₅ | 79 | 240 (Z.) | 1,20 (t, 3 H); 2, 15 (s, 2, 4 H); |
| | | | | | | (A) | | 2,35 (s, 0,6 H); 4,22 (m, 4 H); |
| | | | | | | | | 6, 55 (m, 1 H) ; 7,71 (s, 0,17 H) ; |
| | | | | | | | | 8,20 (s, 0, 83 H) * |
| 3 | CH₃ | CH₃ | H | H | OC₂H₅ | 92 | 200 (Z.) | 1,23 (t, 3 H) ; 1,93 (s, 3 H); |
| | | | | | | (A) | | 2,13 (s, 2,55 H); 2,27 (s, 0,45 H); |
| | | | | | | | | 4,09 (m, 4 H); 6, 39 (t, 0, 82 H) ; |
| | | | | | | | | 6, 48 (t, 0, 18 H) ; 7, 30 (m, 1 H) |
| 4 | CH₃ | H | H | Cl | NHPh | 61 | Öl | 2,34 (m, 3 H); 6,24 (m, 1,2 H); |
| | | | | | | | | 6,86 (m, 0,8 H); |
| | | | | | | | | 7,05 - 7,65 (m, 5,8 H); |
| | | | | | | | | 8,20 (m, 0,6 H); 9,06 (m, 0,6 H) |
| 5 | CH₃ | H | H | Cl | NHC₄H₉ | 58 | Fp: 69-71 | 0,83 (t, 3 H); 1,38 (m, 4 H); |
| | | | | | | | | 2,18 (s, 3 H); 2,98 (m, 2 H); |
| | | | | | | | | 6,22 (m, 1 H) ; 7,00 (s, 1 H) ; |
| | | | | | | | | 7,98 (m, 2 H); *** |
| 6 | CH₃ | H | H | H | OC₂H₅ | 68 | 235 (Z.) | 1,27 (t, 3 H); 2,26 (s, 2,4 H); |
| | | | | | | (B) | | 2,42 (s, 0,6 H) ; 4,14 (m, 4 H); |
| | | | | | | | | 6,06 (d, 1 H); 6,48 (m, 1 H); |
| | | | | | | | | 7,51 (d, 1 H) |
| 7 | CH₃ | H | H | H | OCH₃ | 57 | 237 (Z.) | 2,25 (s, 2,5 H) ; 2,40 (s, 0,5 H) ; |
| | | | | | | (B) | | 3,75 (s, 3H); 4,10 (m, 2 H); |
| | | | | | | | | 6,06 (d, 1 H); 6,45 (m, 1 H) ; |
| | | | | | | | | 7,52 (d, 1 H) |
| 8 | CH₃ | CH₃ | H | H | OCH₃ | 51 | 180-183 | 1,94 (s, 1 H); 2,15 (s, 2,6 H); |
| | | | | | | (A) | | 2,28 (s, 0,4 H); 3,74 (s, 3 H); |
| | | | | | | | | 4,09 (m, 2 H); 6,40 (t, 0,85 H); |
| | | | | | | | | 6,48 (t, 0,15 H); 7,31 (m, 1 H) |
| 9 | CH₃ | H | H | Cl | CH₂CH(CH₃)CH₃ | 67 | 174-176 | 0,94 (m, 6 H); 1,95 (m, 1 H); |
| | | | | | | (B) | | 2,24 (s, 2,9 H) ; 2, 47 (s, 0, 1 H); |
| | | | | | | | | 3,94 (m, 2 H); 6,13 (m, 1 H) ; |
| | | | | | | | | 7,00 (m, 1 H); 7,73 (m, 1 H) |
| 10 | CH₃ | H | H | Cl | Ph | 59 | Öl | 2,33 (m, 3H);6,24 (m, 1H); |
| | | | | | | (B) | | 6,55 (m, 0,5 H) ; 7, 25 (m, 5,5 H) ; |
| | | | | | | | | 7,84 (m, 1 H) |

| | | | | | | | | |
|---|---|---|---|---|---|---|---|---|
| * in DMSO-d₆ aufgenommen | | | | | | | | |

### Ermittlung der nitrifikationshemmenden Wirkung

Die Prüfung der erfindungsgemäßen Pyrazolderivate erfolgte in den in Tabelle 2 ausgewiesenen Konzentrationen. Dabei wurde die nitrifikationshemmende Wirkung in der Weise ermittelt, dass 100 g lufttrockener Boden auf 50 % der maximalen Wasserkapazität eingestellt wurden. Hierzu wurden jeweils 10 mg Harnstoff-N und die ausgewiesene Wirkstoffmenge unter möglichst homogener Verteilung zugesetzt. Aus der Bestimmung des Umsatzes von Ammonium-N zu Nitratstickstoff im Vergleich zur Variante ohne Inhibitor wurde die prozentuale Hemmung zum Tag X ermittelt.

Neben der prozentualen Hemmung wurde der t₅₀-Wert als Maßzahl der Wirksamkeit rechnerisch ermittelt. Der t₅₀-Wert gibt an, in wieviel Tagen 50 % des zugesetzten Amid- bzw. Ammoniumstickstoffs in Nitrat umgewandelt werden.

**Tabelle 2: Nitrifikationshemmende Wirkung ausgewählter Verbindungen**

| **Nr. der Verbindung** | **Wirkstoffkonzentration (ppm)K** | **t**_{**50**}**-Wert (Tage)** |
|---|---|---|
| 1 | 0,5 | 40,73 |
| | 1,0 | 42,49 |
| 2 | 0,5 | 42,7 |
| | 1,0 | 43,1 |
| 3 | 0,5 | 43,2 |
| | 1,0 | 43,7 |
| 4 | 0,5 | 24,9 |
| | 1,0 | 36,9 |
| 5 | 0,5 | 17,6 |
| | 1,0 | 36,4 |
| 6 | 0,5 | 33,4 |
| | 1,0 | 39,3 |
| 7 | 0,5 | 36,8 |
| | 1,0 | 43,2 |
| 8 | 0,5 | 40,0 |
| | 1,0 | 42,2 |
| 9 | 0,5 | 29,8 |
| | 1,0 | 38,8 |
| 10 | 0,5 | 18,0 |
| | 1,0 | 23,4 |

## Patentansprüche

1. 1H-Pyrazol-Derivate der allgemeinen Formel I 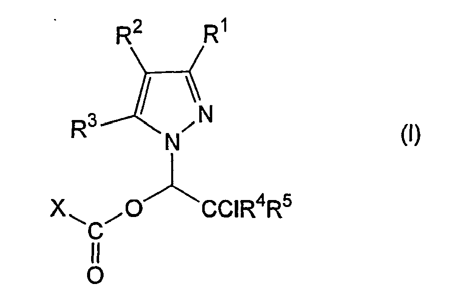 wobei R¹ und R³ unabhängig voneinander für H oder einen C₁-C₄-Alkylrest,
R² für H, Halogen oder einen C₁-C₄-Alkylrest,
R¹ und R² zusammen für einen fünf- oder sechsgliedrigen Cycloalkylrest,
R⁴ und R⁵ unabhängig voneinander für H oder Cl,
und X für OR⁶ oder NHR⁷ stehen,
wobei R⁶ für C₁-C₄-Alkyl, C₆-C₁₀-Aryl oder Alkylaryl mit C₁-C₄-Alkyl- und C₆-C₁₀-Arylgruppen und R⁷ für C₁-C₈-Alkyl oder C₆-C₁₀-Aryl stehen.

2. 1H-Pyrazol-Derivate nach Anspruch 1, **dadurch gekennzeichnet, dass** R¹ und R² unabhängig voneinander einen Methylrest darstellen.

3. 1H-Pyrazol-Derivate nach Anspruch 1, **dadurch gekennzeichnet, dass** R¹ einen Methylrest darstellt und R² für Chlor oder Wasserstoff steht.

4. Verfahren zur Herstellung der 1H-Pyrazol-Derivate mit X = OR⁶ nach einem der Ansprüchen 1 bis 3, **dadurch gekennzeichnet, dass** man die entsprechenden 2-chlorsubstituierten 1-Pyrazolyl-ethanol-Derivate der allgemeinen Formel (IV) 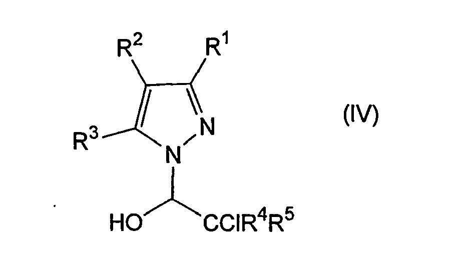 wobei R¹, R², R³, R⁴ und R⁵ oben genannte Bedeutung besitzen, entweder direkt oder gegebenenfalls nach deren Isolierung mit Chlorameisensäureestern Cl-CO-OR⁶, wobei R⁶ die oben genannte Bedeutung besitzt, in einem organischen Lösemittel unter Zusatz von organischen Basen reagieren lässt.

5. Verfahren nach Anspruch 4, **dadurch gekennzeichnet, dass** man die Reaktion in einem organischen Lösemittel ausgewählt aus der Gruppe Halogenkohlenwasserstoffe, cyclische Ether oder aromatische Kohlenwasserstoffe unter Zusatz von molaren Mengen an organischer Base mit einem Molverhältnis von 1-Pyrazolyl-ethanol-Derivat zu Chlorameisensäureester von 1 : 1,0 bis 1,3 und bei Temperaturen von -25 bis +10 °C durchführt.

6. Verfahren zur Herstellung der 1H-Pyrazol-Derivate mit X = NHR⁷ nach einem der Ansprüche 1 bis 3, **dadurch gekennzeichnet, dass** man 2-chlorsubstituierten 1-Pyrazolyl-ethanol-Derivate der allgemeinen Formel (IV) 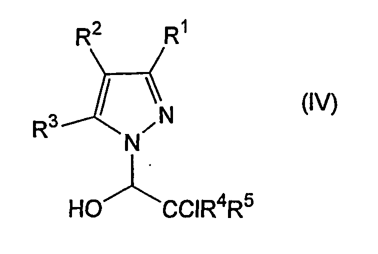 wobei R¹, R², R³, R⁴ und R⁵ oben genannte Bedeutung besitzen, mit Isocyanaten R⁷-N=C=O, wobei R⁷ die oben genannte Bedeutung besitzt, in einem organischen Lösemittel unter Zusatz von organischen Basen umsetzt.

7. Verfahren nach Anspruch 6, **dadurch gekennzeichnet, dass** man die Umsetzung mit den Isocyanaten in einem organischen Lösungsmittel ausgewählt aus der Gruppe Carbonsäureester oder aromatische Kohlenwasserstoffe unter Zusatz von 5 bis 10 Mol% organischer Base mit einem Molverhältnis von 1-Pyrazolyl-ethanol-Derivat (IV) zu Isocyanat von 1 : 1,1 bis 1,3 und bei einer Temperatur von 40 bis 100 °C vornimmt.

8. Verfahren nach den Ansprüchen 4 bis 7, wobei die organische Base Triethylamin ist.

9. Verfahren nach den Ansprüchen 4 bis 8 wobei man zur Herstellung der 2-chlorsubstituierten 1-Pyrazolyl-ethanol-Derivate (IV) die entsprechenden 1-unsubstituierten Basispyrazole der Formel (II), 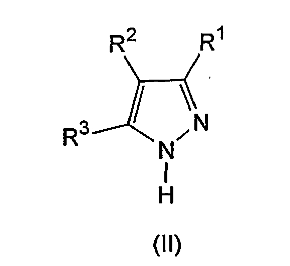 wobei R¹, R² und R³ oben genannte Bedeutung besitzen, mit Aldehyden der allgemeinen Formel (III) 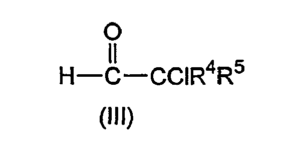 wobei R⁴ und R⁵ die oben genannte Bedeutung besitzen, im Molverhältnis 1 : 1,0 bis 1 : 1,1 in Gegenwart eines organischen Lösemittels bei Temperaturen von 50 bis 80 °C zur Reaktion umsetzt.

10. Verwendung der 1H-Pyrazol-Derivate nach den Ansprüchen 1 bis 3 zur Hemmung bzw. Steuerung der Nitrifikation.

11. Zusammensetzung umfassend die 1H-Pyrazol-Derivate nach den Ansprüchen 1 bis 3 sowie gegebenenfalls weitere übliche Nitrifiaktionsinhibitoren und/oder Ureasehemmstoffe in Kombination mit festen und flüssigen ammonium- und/oder amidhaltigen Düngemitteln.

12. Zusammensetzung nach Anspruch 11, **dadurch gekennzeichnet, dass** die 1H-Pyrazol-Derivate sowie die gegebenenfalls weiteren üblichen Nitrifiaktionsinhibitoren und/oder Ureasehemmstoffe in einer Gesamtaufwandmenge von 0,01 bis 10,0 Gewichtsprozent berechnet auf den reduzierten Stickstoffanteil des amid- und/oder ammoniumhaltigen Düngemittels, eingesetzt werden.

13. Zusammensetzung nach Anspruch 12, **dadurch gekennzeichnet dass** die 1H-Pyrazol-Derivate in einer Aufwandmenge von 0,3 bis 3,0 Gewichtsprozent eingesetzt werden.

14. Zusammensetzung nach den Ansprüchen 11 bis 13, **dadurch gekennzeichnet, dass** die 1H-Pyrazol-Derivate allein oder im Gemisch mit weiteren Zusatzstoffen in die Düngemittelschmelze oder -slurry während des Formgebungsprozesses eingebracht worden sind und auf diese Weise homogen im Dünger verteilt vorliegen.

15. Zusammensetzung nach den Ansprüchen 11 bis 13, **dadurch gekennzeichnet, dass** die 1H-Pyrazol-Derivate ggf. mit weiteren Zusatzstoffen in Form einer Lösung auf die Oberfläche der Düngemittelgranulate aufgebracht worden sind und anschließend das Lösemittel durch Trocknung entfernt wurde.

## Claims

1. 1H-pyrazole derivatives with the general formula I 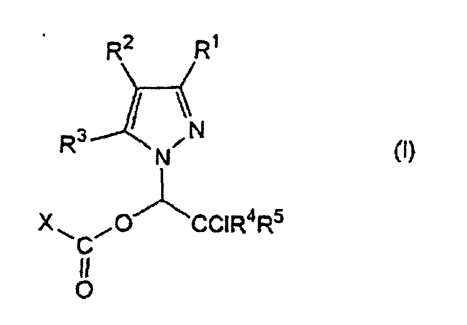
R¹ and R³ standing independently of one another for H or a C₁-C₄-alkyl residue, R² for H, halogen or a C₁-C₄-alkyl residue,
R¹ and R² together for a five- or six-membered cycloalkyl residue,
R⁴ and R⁵ independently of one another for H or Cl,
and X for OR⁶ or NHR⁷,
R⁶ standing for C₁-C₄-alkyl, C₆-C₁₀-aryl or alkylaryl with C₁-C₄-alkyl and C₆-C₁₀₋aryl groups, and
R⁷ for C₁-C₈-alkyl or C₆-C₁₀-aryl.

2. 1H-pyrazole derivatives according to Claim 1, **characterised in that** R¹ and R² represent, independently of one another, a methyl residue.

3. 1H-pyrazole derivatives according to Claim 1, **characterised in that** R¹ represents a methyl residue, and R² stands for chlorine or hydrogen.

4. A method for producing the 1H-pyrazole derivatives with X = OR⁶ according to any of Claims 1 to 3, **characterised in that** the corresponding 2-chlorine-substituted 1-pyrazolyl-ethanol-derivatives with the general formula (IV) 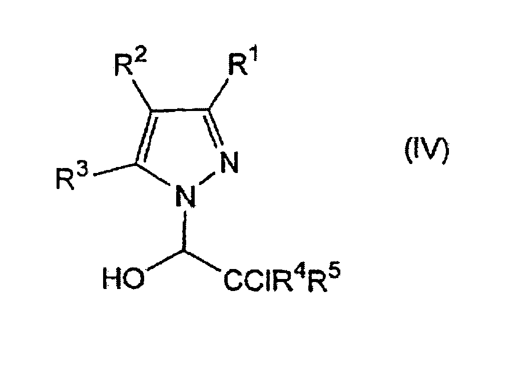 R¹, R², R³, R⁴ and R⁵ having the aforementioned meaning, are allowed to react in an organic solvent with the addition of organic bases, either directly or if appropriate following the isolation of the same with chloroformic acid esters Cl-CO-OR⁶, R⁶ having the aforementioned significance.

5. The method according to Claim 4, **characterised in that** one implements the reaction in an organic solvent selected from the group of halogenated hydrocarbons, cyclic ethers or aromatic hydrocarbons, with the addition of molar quantities of organic base with a mole ratio of 1-pyrazolyl-ethanol-derivative to chloroformic acid ester of 1 : 1.0 to 1.3, and at temperatures of between -25 and +10°C.

6. A method for producing the 1H-pyrazole derivatives with X = NHR⁷ according to any of Claims 1 to 3, **characterised in that** 2-chlorine-substituted 1-pyrazolyl-ethanol-derivatives with the general Formula (IV) 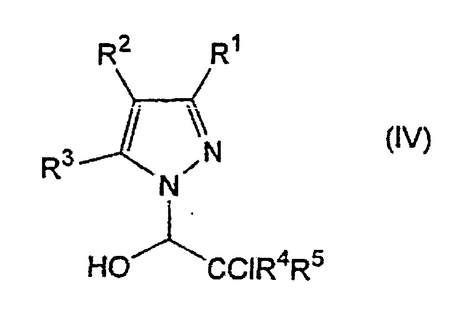 are converted in an organic solvent with the addition of organic bases, R¹, R², R³, R⁴ and R⁵ having the aforementioned meaning, with isocyanates R⁷-N=C=0, R⁷having the aforementioned meaning.

7. The method according to Claim 6, **characterised in that** the conversion is undertaken with the isocyanates in an organic solvent selected from the group of carboxylic acid esters or aromatic hydrocarbons with the addition of 5 to 10 moles % organic base with a mole ratio of 1-pyrazolyl-ethanol-derivative (IV) to isocyanate of 1 : 1.1 to 1.3, and at a temperature of 40 to 100°C.

8. The method according to Claims 4 to 7, the organic base being triethylamine.

9. The method according to Claims 4 to 8, in order to produce the 2-chlorine-substituted 1-pyrazolyl-ethanol derivatives (IV), the corresponding 1-unsubstituted base pyrazoles with the formula (II), 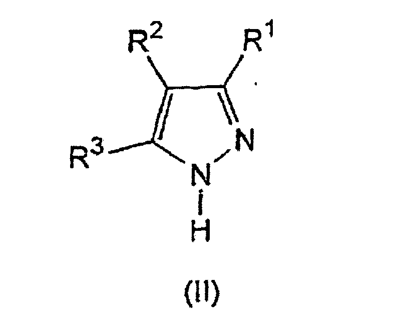 being brought to react in the presence of an organic solvent at temperatures of between 50 and 80°C, R¹, R² and R³ having the aforementioned meaning, with aldehydes with the general formula (III) 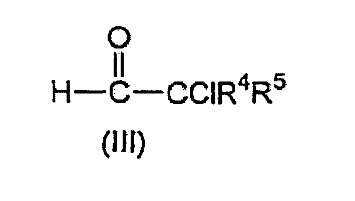 R⁴ and R⁵ having the aforementioned meaning, and in the mole ratio 1 : 1.0 to 1 : 1.1.

10. Use of the 1H-pyrazole derivatives according to Claims 1 to 3 in order to inhibit or control nitrification.

11. A composition comprising the 1H-pyrazole derivatives according to Claims 1 to 3 and if appropriate further conventional nitrification inhibitors and/or urea inhibitors in combination with solid and liquid fertilisers containing ammonium and/or amide.

12. The composition according to Claim 11, **characterised in that** the 1H-pyrazole derivatives and if appropriate the further conventional nitrification inhibitors and/or urea inhibitors are used at a total application rate of 0.01 to 10.0 weight per cent calculated to the reduced nitrogen portion of the fertiliser containing the amide and/or ammonium.

13. The composition according to Claim 12, **characterised in that** the 1H-pyrazole derivatives are used at an application rate of between 0.3 and 3.0 weight percent.

14. The composition according to Claims 11 to 13, **characterised in that** the 1 H-pyrazole derivatives on their own or in a mixture with further additives were added to the fertiliser melt or slurry during the forming process and in this way are provided distributed homogeneously in the fertiliser.

15. The composition according to Claims 11 to 13, **characterised in that** the 1 H-pyrazole derivatives are applied if appropriate with further additives in the form of a solution onto the surface of the fertiliser pellets, and then the solvent is removed by drying.

## Revendications

1. Dérivés de 1H-pyrazole répondant à la formule générale I 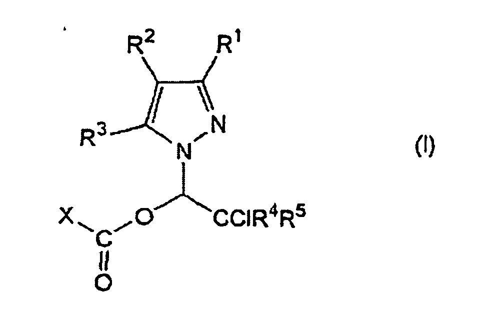
dans laquelle R¹ et R³ représentent, indépendamment l'un de l'autre, H ou un reste alkyle en C₁ à C₄,
R² représente H, un halogène ou un reste alkyle en C₁ à C₄,
R¹ et R² représentent ensemble un reste cycloalkyle à cinq ou six membres,
R⁴ et R⁵ représentent, indépendamment l'un de l'autre, H ou Cl,
Et X représente OR⁶ ou NHR⁷,
dans lesquels R⁶ représente un groupe alkyle en C₁ à C₄, aryle en C₆ à C₁₀ ou alkylaryle avec des groupes alkyle en C₁ à C₄ et aryle en C₆ à C₁₀ et R⁷ représente un groupe alkyle en C₁ à C₈ ou aryle en C₆ à C₁₀.

2. Dérivés de 1H-pyrazole selon la revendication 1, **caractérisés en ce que** R¹ et R² représentent indépendamment l'un de l'autre un reste méthyle.

3. Dérivés de 1H-pyrazole selon la revendication 1, **caractérisés en ce que** R¹ représente un reste méthyle et R² représente le chlore ou l'hydrogène.

4. Procédé de production de dérivés de 1H-pyrazole dans lesquels X = R⁶ selon l'une des revendications 1 à 3, **caractérisé en ce que** l'on fait réagir, dans un solvant organique additionné de bases organiques, les dérivés de 1-pyrazolyl-éthanol à substitution 2-chlore appropriés de la formule générale (IV) 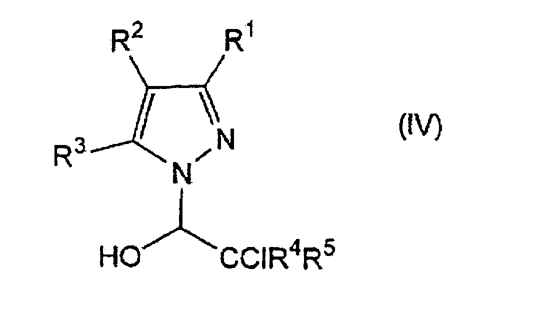 dans laquelle R¹, R², R³, R⁴ et R⁵ ont la signification donnée ci-dessus, soit directement soit éventuellement après leur isolation par des esters d'acide chloroformique Cl-CO-OR⁶, où R⁶ a la signification donnée ci-dessus.

5. Procédé selon la revendication 4, **caractérisé en ce qu'**on conduit la réaction dans un solvant organique choisi dans le groupe constitué par les hydrocarbures halogénés, les éthers cycliques ou les hydrocarbures aromatiques en ajoutant des quantités molaires d'une base organique dans un rapport molaire de dérivé de 1-pyrazolyl-éthanol : ester d'acide chloroformique allant de 1 : 1,0 à 1,3 et à des températures allant de -25 à +10 °C.

6. Procédé de production de dérivés de 1H-pyrazole dans lesquels X = NHR⁷ selon l'une des revendications 1 à 3, **caractérisé en ce que** l'on fait réagir, dans un solvant organique additionné de bases organiques, les dérivés de 1-pyrazolyl-éthanol à substitution 2-chlore appropriés de la formule générale (IV) 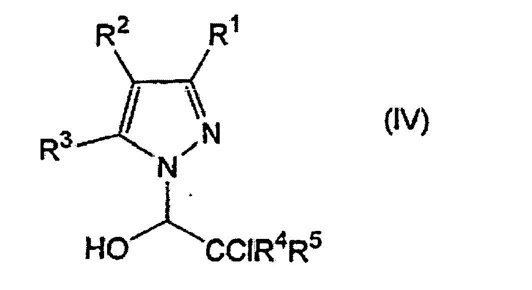 dans laquelle R¹, R², R³, R⁴ et R⁵ ont la signification donnée ci-dessus, avec les isocyanates R⁷-N=C=O, où R⁷ a la signification donnée ci-dessus.

7. Procédé selon la revendication 6, **caractérisé en ce qu'**on entreprend la réaction avec les isocyanates dans un solvant organique choisi dans le groupe constitué par les esters carboxyliques ou les hydrocarbures aromatiques en ajoutant de 5 à 10 % en mole d'une base organique dans un rapport molaire de dérivé d'éthanol de pyrazolyle (IV) : isocyanate allant de 1 : 1,1 à 1,3 et à une température allant de 40 à 100 °C.

8. Procédé selon les revendications 4 à 7, dans lequel la base organique est de la triéthylamine.

9. Procédé selon les revendications 4 à 8, dans lequel, pour produire les dérivés de 1-pyrazolyl-éthanol (IV) à substitution 2-chlore, on fait réagir les pyrazoles de base 1-substitués appropriés répondant à la formule (II) 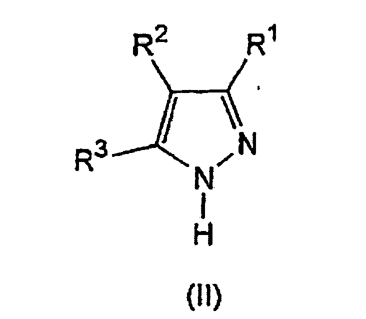 dans laquelle R¹, R² et R³ ont la signification donnée ci-dessus, avec des aldéhydes de la formule générale (III) 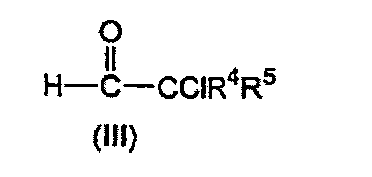 dans laquelle R⁴ et R⁵ ont la signification donnée ci-dessus, dans un rapport molaire de 1 : 1,0 à 1 : 1,1 en présence d'un solvant organique à des températures allant de 50 à 80 °C.

10. Utilisation des dérivés de 1H-pyrazole selon les revendications 1 à 3 pour inhiber ou réguler la nitrification.

11. Composition comprenant les dérivés de 1H-pyrazole selon les revendications 1 à 3 ainsi qu'éventuellement d'autres inhibiteurs de nitrification courants et/ou d'inhibiteurs de l'uréase en combinaison avec des engrais solides ou liquides contenant de l'ammonium et/ou des amides.

12. Composition selon la revendication 11, **caractérisée en ce que** les dérivés de 1H-pyrazole ainsi que les éventuels autres inhibiteurs de nitrification courants et/ou inhibiteurs de l'uréase sont employés suivant une dose d'emploi totale allant de 0,01 à 10,0 pour cent en poids par rapport à la proportion d'azote réduite de l'engrais contenant de l'ammonium et/ou des amides.

13. Composition selon la revendication 12, **caractérisée en ce que** les dérivés de 1H-pyrazole sont employés suivant une dose d'emploi allant de 0,3 à 3,0 pour cent en poids.

14. Composition selon les revendications 11 à 13, **caractérisée en ce que** les dérivés de 1H-pyrazole sont introduits seuls ou en mélange avec des additifs dans la masse fondue ou la suspension d'engrais durant le procédé de formage en étant ainsi répartis de manière homogène dans l'engrais.

15. Composition selon les revendications 11 à 13, **caractérisée en ce que** les dérivés de 1H-pyrazole, ainsi que leurs éventuels additifs, ont été appliqués sous la forme d'une solution sur la surface de granulés d'engrais avant d'éliminer le solvant par évaporation.
